# FASCICULE DE BREVET EUROPEEN

(11) **EP 3 989 988 B1**
(45) Date de publication et mention de la délivrance du brevet: **02.08.2023**
(21) Numéro de dépôt: 20739275.4
(22) Date de dépôt: 24.06.2020
(51) Int. Cl.: A61K 35/74, A61K 35/741, A61P 13/12, A61K 45/06

(54) **BACTERIE DE LA FAMILLE DES CHRISTENSENELLACEES ET COMPOSITION EN CONTENANT POUR LA PREVENTION ET/OU LE TRAITEMENT DE L'INSUFFISANCE RENALE**
BAKTERIUM DER FAMILIE CHRISTENSENELLACEAE UND DIESE ENTHALTENDE ZUSAMMENSETZUNG ZUR VORBEUGUNG UND/ODER BEHANDLUNG VON NIERENINSUFFIZIENZ
BACTERIUM OF THE CHRISTENSENELLACEAE FAMILY AND COMPOSITION CONTAINING SAME FOR PREVENTING AND/OR TREATING RENAL INSUFFICIENCY

(30) Priorité: 25.06.2019 FR 1906842
(43) Date de publication de la demande: 04.05.2022
(73) Titulaire: YSOPIA Biosciences, 33076 Bordeaux Cedex (FR)
(72) Inventeur: RAWADI, Georges, 75011 PARIS (FR); CLAUS, Sandrine, 33200 BORDEAUX (FR); RINALDI, Laure, 33000 BORDEAUX (FR)
(74) Mandataire: Aquinov
(86) Numéro de dépôt international: PCT/EP2020/067630
(87) Numéro de publication internationale: WO 2020/260356

(56) Documents cités:
- US-A1- 2002 187 134
- US-A1- 2004 106 185
- US-A1- 2004 197 352
- US-A1- 2017 042 948
- CHIH-JUNG CHANG ET AL: "Next generation probiotics in disease amelioration", JOURNAL OF FOOD AND DRUG ANALYSIS, vol. 27, no. 3, 2 février 2019 (2019-02-02), pages 615-622, XP055649488, TW ISSN: 1021-9498, DOI: 10.1016/j.jfda.2018.12.011
- KANBAY MEHMET ET AL: "The crosstalk of gut microbiota and chronic kidney disease: role of inflammation, proteinuria, hypertension, and diabetes mellitus", INTERNATIONAL UROLOGY AND NEPHROLOGY, AKADEMIAI, BUDAPEST, HU, vol. 50, no. 8, 4 mai 2018 (2018-05-04), pages 1453-1466, XP036561754, ISSN: 0301-1623, DOI: 10.1007/S11255-018-1873-2 [extrait le 2018-05-04]
- PAN WEI ET AL: "Gut microbiota and chronic kidney disease: implications for novel mechanistic insights and therapeutic strategies", INTERNATIONAL UROLOGY AND NEPHROLOGY, AKADEMIAI, BUDAPEST, HU, vol. 50, no. 2, 28 août 2017 (2017-08-28), pages 289-299, XP036421947, ISSN: 0301-1623, DOI: 10.1007/S11255-017-1689-5 [extrait le 2017-08-28]
- KHODOR SOUHAILA AL ET AL: "Gut microbiome and kidney disease: a bidirectional relationship", PEDIATRIC NEPHROLOGY, SPRINGER VERLAG, BERLIN, DE, vol. 32, no. 6, 29 avril 2016 (2016-04-29) , pages 921-931, XP036216076, ISSN: 0931-041X, DOI: 10.1007/S00467-016-3392-7 [extrait le 2016-04-29]

## Description

### DOMAINE TECHNIQUE

L'invention concerne la prévention et le traitement de l'insuffisance rénale. En particulier l'invention se rapporte à des bactéries spécifiques du microbiote intestinal et des compositions en contenant pour la prévention et/ou le traitement de l'insuffisance rénale.

### ART ANTERIEUR

L'insuffisance rénale est une maladie dont la pathophysiologie est directement liée à la destruction des cellules du rein. Cette destruction des cellules rénales conduisant à une insuffisance rénale est provoquée par une néphropathie. Il existe différents types de néphropathies, telles que notamment les néphropathies vasculaires et hypertensives, les néphropathies diabétiques essentiellement avec un diabète de type 2, les glomérulonéphrites chroniques, les néphropathies héréditaires essentiellement polykystose rénale autosomique dominante, les néphropathies interstitielles chroniques, les néphropathies diverses et les néphropathies d'origine indéterminée.

L'insuffisance rénale chronique touche une large portion de la population. Les conséquences pour la santé sont majeures, car il s'agit d'une pathologie invalidante, douloureuse et associée à un risque élevé de mortalité précoce.

Actuellement, le traitement de l'insuffisance rénale chronique repose :
- sur la prise en charge de la maladie d'origine de l'insuffisance rénale (hypertension artérielle, diabète, polykystose, etc.) qui est souvent à un stade très avancé lors d'apparition d'insuffisance rénale chronique,
   - sur le ralentissement de l'évolution vers l'insuffisance rénale chronique en protégeant les reins,
   - sur des interventions destinées à ralentir la progression des maladies rénales chroniques notamment par le contrôle strict de la pression artérielle, par la diminution de la protéinurie, par l'utilisation d'inhibiteurs de l'enzyme de conversion ou de bloqueurs des récepteurs de type 1 de l'angiotensine II, par la prévention des épisodes d'insuffisance rénale aiguë et de la néphrotoxicité, par la restriction protidique modérée et adaptée au patient, par le contrôle d'un diabète s'il existe et l'arrêt du tabac,
   - sur la dialyse et la greffe des reins si le stade d'insuffisance rénale terminale est atteint.

Toutefois, ces différents traitements ont des taux de réponses très variable, en particulier du fait du nombre important de facteurs intervenant dans l'apparition de l'insuffisance rénale, notamment l'insuffisance rénale chronique, et du fait de la dégradation des molécules lorsqu'elles sont administrées. En outre ces traitements présentent des effets secondaires importants tels que notamment : maux de tête, nausées, fatigue, hypoglycémie, rétention hydrosodée, effets secondaires digestifs (flatulences), hypotension, perte de muscle, une hyperkaliémie, oedème angioneurotique, foetotoxicité, crampes, troubles du sommeil, démangeaisons, sténoses, thrombose, lymphocèle, hématome, tubulonéphrite aiguë, rejet de greffe, diabète, augmentation du risque de cancer, risque de maladies cardio-vasculaires, maladies hépatiques.

Ainsi, il existe un besoin important pour un traitement efficace de l'insuffisance rénale, notamment de l'insuffisance rénale chronique, qui fonctionne quel que soit le facteur d'origine de la maladie, qui soit facile à administrer, et qui ne présente pas d'effets secondaires.

### EXPOSE DE L'INVENTION

C'est l'objectif de la présente invention, qui, pour y répondre, vise l'utilisation de bactéries particulières du microbiote intestinal humain, à savoir des bactéries de la famille des *Christensenellacées.*

Des bactéries de la famille des *Christensenellacées,* notamment du genre *Christensenella,* ont déjà été étudiées et décrites. C'est le cas en particulier de *Christensenella minuta, Christensenella massiliensis* et *Christensenella timonensis. Christensenella minuta* en particulier a été décrite pour la première fois en 2012. En 2014, une étude a montré qu'il s'agissait du taxon le plus héritable dans une cohorte de jumeaux britanniques et que leur présence est associée à un indice de masse corporel faible. Cette corrélation entre *Christensenella minuta* et l'indice de masse corporel faible a ensuite été observée dans une dizaine d'études parues depuis 2014 dans des populations géographiquement diverses.

De façon surprenante, selon l'invention, les bactéries de la famille des *Christensenellacées,* lorsqu'elles sont administrées à l'homme ou à l'animal, sont capables d'agir sur les marqueurs de l'insuffisance rénale pour traiter cette maladie.

C'est pourquoi, l'invention a pour objet une bactérie de la famille des *Christensenellacées,* pour son utilisation dans la prévention et/ou le traitement de l'insuffisance rénale chez l'être humain ou l'animal. Il peut s'agir d'une insuffisance rénale aiguë ou chronique, préférentiellement d'une insuffisance rénale chronique.

Avantageusement, une telle bactérie, lorsqu'elle est administrée à un être humain ou un animal présentant une insuffisance rénale, est capable d'agir sur les molécules produites en excès lors de cette maladie comme en particulier le CTGF urinaire, l'interleukine 18 du tissu rénal, l'apolipoprotéine A-IV plasmatique, les cellules mononucléaires CD14 urinaires, la molécule-1 de l'insuffisance rénale sérique, le facteur de croissance de fibroblaste-23, la protéine liant les acides gras du foie urinaire, la glucosaminidase N-acetyl-b-O urinaire, la gélatinase neutrophile associée à la lipocaline, la protéine 4 liant le rétinol urinaire, l'homocitrulline sérique, la dimethylarginine symétrique sérique. En outre, il s'agit d'une bactérie naturellement présente dans le microbiote intestinal, et dont l'administration ne provoque pas d'effets secondaires contrairement aux traitements existants.

Préférentiellement les bactéries pour leur utilisation selon l'invention sont administrées au sein de compositions. Aussi, l'invention a également pour objet les compositions comprenant au moins une bactérie de la famille des *Christensenellacées,* pour son utilisation dans la prévention et/ou le traitement de l'insuffisance rénale chez l'être humain ou l'animal.

D'autres caractéristiques et avantages ressortiront de la description en détails de l'invention qui va suivre.

### DESCRIPTION DETAILLEE DE L'INVENTION

### Définitions

Par « hyperproduction » ou « production en excès » d'une molécule ou d'un marqueur au sens de l'invention on entend une production excessive de ladite molécule ou dudit marqueur par rapport à la production chez une personne ou un animal sain sans insuffisance rénale.

Par « marqueur » d'une maladie au sens de l'invention on entend une molécule ou une substance dont le dosage permet de suivre l'évolution de ladite maladie.

### Bactéries selon l'invention

L'invention a pour objet l'utilisation pour prévenir et/ou traiter l'insuffisance rénale aiguë ou chronique, préférentiellement l'insuffisance rénale chronique, chez l'être humain ou l'animal, d'au moins une bactérie de la famille des *Christensenellacées.*

L'invention vise donc une bactérie de la famille des *Christensenellacées* pour son utilisation dans la prévention et/ou le traitement de l'insuffisance rénale chez l'être humain ou l'animal, en particulier chez des personnes ou des animaux présentant une insuffisance rénale, notamment chronique, avec une hyperproduction d'au moins un marqueur choisi parmi : le CTGF urinaire, l'interleukine 18 du tissu rénal, la béta-microglobuline, l'alpha1-microglobuline, la microalbumine, l'apolipoprotéine A-IV plasmatique, les cellules mononucléaires CD14 urinaires, la clustérine, la molécule-1 de l'insuffisance rénale sérique, le facteur de croissance de fibroblaste-23, la protéine liant les acides gras du foie urinaire, la glucosaminidase N-acetyl-b-O urinaire, la gélatinase neutrophile associée à la lipocaline, l'ostéopontine, la protéine 4 liant le rétinol urinaire, l'homocitrulline sérique, la dimethylarginine symétrique sérique.

Le CTGF (facteur de croissance du tissu conjonctif) urinaire est facteur profibrotique important dans les maladies rénales, dont le blocage augmente les dommages rénaux (Sánchez-Ló pez, E. et al. CTGF Promotes Inflammatory Cell Infiltration of the Renal Interstitium by Activating NF-B. J Am Soc Nephrol 20, 1513-1526 (2009)).

L'interleukine 18 est un médiateur de la nécrose tubulaire aiguë ischémique et a été montré comme marqueur rapide et fiable pour la détection précoce de l'insuffisance rénale, notamment l'insuffisance rénale aiguë (Lin, X., Yuan, J., Zhao, Y. & Zha, Y. Urine interleukin-18 in prédiction of acute kidney injury: a systemic review and meta-analysis. J. Nephrol. 28, 7-16 (2015)).

L'aphal-microglobuline est un marqueur de dysfonction tubulaire proximale, dès la phase précoce de lésion lorsque aucun dommage histologique est observé ; la microalbumine est un marqueur de dommages des cellules tubulaires ; la béta -microglobuline est un marqueur excrété lors de lésions tubulaires ; la clusterine et la molécule-1 sont des marqueurs servant dans le diagnostic précoce de lésions rénales et toxicité tubulaire proximale ; les protéines liant les acides gras du foie urinaire (la L-FABP dans le tubule proximal et la H-FABP dans le tubule distal) sont des marqueurs de lésions tubulo-intersticiels ; la glucosamine N-acetyl-b-O urinaire est un marqueur sensible, persistant et robuste indiquant une lésion tubulaire ; la gélatinase neutrophile est un marqueur de l'insuffisance rénale lorsque celle-ci est liée à des conditions inflammatoires ou infectieuses ; l'ostéopontine est un marqueur surexprimé dans des biopsies rénales de patients avec hypertension_Cardenas-Gonzalez, M., Pavkovic, M. & Vaidya, V. S. Biomarkers of Acute Kidney Injury. in Comprehensive Toxicology: Third Edition 14-15, 147-163 (Elsevier Inc., 2017)).

L'apolipoprotéine A-IV plasmatique est un marqueur issu des entérocytes intestinales montrant la progression de la maladie (Boes, E. et al. Apolipoprotein A-IV Predicts Progression of Chronic Kidney Disease: The Mild to Moderate Kidney Disease Study. J. Am. Soc. Nephrol. 17, 528-536 (2006)).

Les cellules mononucléaires CD14 urinaires sont des marqueurs d'insuffisance rénale positivement corrélés avec le volume du rein et liés à la maladie polykystique du rein ; le facteur de croissance de fibroblaste-23 est un marqueur d'origine dans les ostéocytes ou les ostéoblastes, montrant la progression des maladies rénales et de la mortalité ; la protéine 4 liant le rétinol urinaire est un marqueur du tubule proximal dont la présence est liée à une dysfonction tubulaire proximale (Lopez-Giacoman, S. Biomarkers in chronic kidney disease, from kidney function to kidney damage. World J. Nephrol. 4, 57 (2015)).

L'homocitrulline sérique est un produit dérivé de la carbamylation et est donc un marqueur de morbidité et mortalité de l'insuffisance rénale (Jaisson, S. et al. Homocitrulline as marker of protein carbamylation in hemodialyzed patients. Clin. Chim. Acta 460, 5-10 (2016)).

La diméthylarginine symétrique sérique est un marqueur extrait de l'artère rénale montrant un dommage de l'endothélium, souvent lié à l'hypertension d'un patient souffrant d'insuffisance rénale (Fleck, C., Schweitzer, F., Karge, E., Busch, M. & Stein, G. Serum concentrations of asymmetric (ADMA) and symmetric (SDMA) dimethylarginine in patients with chronic kidney diseases. Clin. Chim. Acta 336, 1-12 (2003) et Nijveldt, R. J. et al. Handling of asymmetrical dimethylarginine and symmetrical dimethylarginine by the rat kidney under basal conditions and during endotoxaemia. Nephrol. Dial. Transplant. 18, 2542-2550 (2003)).

Selon l'invention, les bactéries de la famille des *Christensenellacées,* lorsqu'elles sont administrées à un être humain ou un animal présentant une insuffisance rénale, notamment une insuffisance rénale chronique, sont capables d'agir sur les molécules produites en excès lors d'une insuffisance rénale, notamment lors d'une insuffisance rénale chronique, en particulier sur la production d'au moins un marqueur choisi parmi : le CTGF urinaire, l'interleukine 18 du tissu rénal, la béta-microglobuline, l'alpha1-microglobuline, la microalbumine, l'apolipoprotéine A-IV plasmatique, les cellules mononucléaires CD14 urinaires, la clustérine, la molécule-1 de l'insuffisance rénale sérique, le facteur de croissance de fibroblaste-23, la protéine liant les acides gras du foie urinaire, la glucosaminidase N-acetyl-b-O urinaire, la gélatinase neutrophile associée à la lipocaline, l'ostéopontine, la protéine 4 liant le rétinol urinaire, l'homocitrulline sérique, la dimethylarginine symétrique sérique.

Dans les insuffisances rénales, notamment chroniques, qui présentent une augmentation d'au moins un marqueur choisi parmi : le CTGF urinaire, l'interleukine 18 du tissu rénal, la béta-microglobuline, l'alpha1-microglobuline, la microalbumine, l'apolipoprotéine A-IV plasmatique, les cellules mononucléaires CD14 urinaires, la clustérine, la molécule-1 de l'insuffisance rénale sérique, le facteur de croissance de fibroblaste-23, la protéine liant les acides gras du foie urinaire, la glucosaminidase N-acetyl-b-O urinaire, la gélatinase neutrophile associée à la lipocaline, l'ostéopontine, la protéine 4 liant le rétinol urinaire, l'homocitrulline sérique, la dimethylarginine symétrique sérique, leur diminution est le signe de la réduction des voies de signal de dégradation des cellules rénales, c'est-à-dire que les cellules rénales à l'origine de cette production sont moins stimulées et que la carbamylation des protéines est moins stimulée. Dès lors, la dégradation excessive des cellules en cause de l'insuffisance rénale, notamment l'insuffisance rénale chronique, est ralentie et le système retourne progressivement à la norme.

Les bactéries utiles selon l'invention sont administrées à des êtres humains ou des animaux dans une quantité efficace pour une action sur au moins l'un de ces marqueurs de l'insuffisance rénale chronique, c'est-à-dire pour diminuer la production d'au moins un de ces marqueurs dans l'organisme.

Selon un mode de réalisation adapté, la ou les bactéries sont administrées à raison d'une dose de 10⁹ à 10¹² unités formant des colonies (CFU) par jour, quel que soit le poids de la personne ou de l'animal. Préférentiellement il s'agit d'une dose unique, c'est-à-dire administrée en une seule fois ou une dose avant chaque repas soit trois fois par jour.

Les bactéries utiles selon l'invention sont des bactéries de la famille des *Christensenellacées,* préférentiellement du genre *Christensenella.* Il peut s'agir en particulier de *Christensenella massiliensis, Christensenella timonensis* et/ou *Christensenella minuta.* Selon une variante particulièrement adaptée, il s'agit de *Christensenella minuta.*

Ces bactéries peuvent être isolées à partir de selles humaines par exemple selon les protocoles publiés par Morotomi et al. 2012 (Morotomi, M., Nagai, F. & Watanabe, Y. Description of Christensenella minuta gen. nov., sp. nov., isolated from human faeces, which forms a distinct branch in the order Clostridiales, and proposal of Christensenellaceae fam. nov. INTERNATIONAL JOURNAL OF SYSTEMATIC AND EVOLUTIONARY MICROBIOLOGY 62, 144-149 (2012)) et NDongo et al. 2016 (Ndongo, S., Dubourg, G., Khelaifia, S., Fournier, P. E. & Raoult, D. Christensenella timonensis, a new bacterial species isolated from the human gut. New Microbes and New Infections 13, 32-33 (2016)). Ces documents décrivent également les méthodes de culture des bactéries utiles selon l'invention.

### Compositions

Les bactéries utiles selon l'invention, sont préférentiellement administrées à l'être humain ou l'animal dans une composition.

Ainsi, l'invention a également pour objet une composition comprenant au moins une bactérie de la famille des *Christensenellacées* pour son utilisation dans la prévention et/ou le traitement de l'insuffisance rénale, notamment l'insuffisance rénale chronique, chez l'être humain ou l'animal, notamment chez des personnes ou des animaux présentant une hyperproduction d'au moins un marqueur choisi parmi : le CTGF urinaire, l'interleukine 18 du tissu rénal, la béta-microglobuline, l'alpha1-microglobuline, la microalbumine, l'apolipoprotéine A-IV plasmatique, les cellules mononucléaires CD14 urinaires, la clustérine, la molécule-1 de l'insuffisance rénale sérique, le facteur de croissance de fibroblaste-23, la protéine liant les acides gras du foie urinaire, la glucosaminidase N-acetyl-b-O urinaire, la gélatinase neutrophile associée à la lipocaline, l'ostéopontine, la protéine 4 liant le rétinol urinaire, l'homocitrulline sérique, la dimethylarginine symétrique sérique.

Les bactéries sont présentes en une quantité efficace dans la composition permettant un effet sur l'insuffisance rénale, notamment l'insuffisance rénale chronique, dont sont atteints les personnes ou les animaux traités.

Préférentiellement, la composition utile selon l'invention comprend 10⁶ à 10¹² unités formant des colonies (CFU) de bactéries de la famille des *Christensenellacées* par dose quotidienne de composition à administrer. Préférentiellement cela correspond à une dose quotidienne de bactéries à administrer, quel que soit le poids de la personne ou de l'animal. De façon préférée, cette dose quotidienne est administrée en une seule fois.

La composition utile selon l'invention peut être sous forme liquide. Elle peut notamment comprendre des bactéries de la famille des *Christensenellacées* et un milieu de culture desdites bactéries qui permet de les conserver, comme par exemple préférentiellement le milieu Columbia agar anaérobique enrichi en sang de mouton, ou un milieu équivalent ne contenant pas de produit dérivé d'origine animale.

Lorsque les compositions sont sous forme liquide, elles sont préférentiellement congelées, maintenues à -20°C dans un sachet hermétique.

Selon une variante, la composition utile selon l'invention peut se présenter sous forme solide. Dans ce cas les bactéries peuvent être présentes sous forme lyophilisée, et les compositions peuvent comprendre également des excipients tels que par exemple la cellulose microcrystalline, le lactose, le saccharose, le fructose, le lévulose, les amidons, le stachyose, le raffinose, l'amylum, le lactate de calcium, le sulfate de magnésium, le citrate de sodium, le calcium stéarate, la polyvinylpyrrolidone, la maltodextrine, les galactooligosaccharides, les fructooligosaccharides, les pectines, les béta-glucans, les lactoglobulines, les isomaltooligosaccharides, les polydextroses, le sorbitol et/ou le glycérol.

Les compositions utiles selon l'invention peuvent se présenter en particulier sous forme de poudre, de poudre microencapsulée, de gélule, de capsule, de comprimé, de pastille, de granulés, d'émulsion, de suspension ou de suppositoire. Selon un mode de réalisation particulièrement adapté, elles peuvent se présenter sous une forme gastro-résistante, telles qu'un comprimé enrobé contenant des bactéries microencapsulées.

Lorsque les compositions sont sous forme solide, elles sont préférentiellement conditionnées dans des capsules ou dans un enrobage hermétique à la lumière et à l'oxygène maintenu à une température ambiante comprise entre 15°C et 40°C et un taux d'humidité compris entre 3% et 70%.

Les bactéries peuvent être utilisées vivantes ou inactivées par exemple par la chaleur, l'exposition à un pH approprié, aux rayons gamma ou à la mise sous haute pression.

Elles peuvent être toutes vivantes ou toutes inactivées.

Préférentiellement, au moins une partie des bactéries sont vivantes, en particulier au moins 50% (en nombre), encore plus préférentiellement au moins 90% (en nombre).

Ainsi, selon un mode de réalisation adapté, les bactéries présentes dans la composition utile selon l'invention sont pour au moins 50% des bactéries vivantes (en nombre), préférentiellement pour au moins 90% des bactéries vivantes (en nombre), encore plus préférentiellement toutes vivantes.

Les bactéries utiles selon l'invention, et en particulier les compositions l'incluant, peuvent être administrées par voie orale, topique, respiratoire (inhalation) ou rectale.

Les compositions utiles selon l'invention, en plus des bactéries utiles selon l'invention peuvent comprendre d'autres composés, tels que :
- au moins un probiotique, et/ou
- au moins une bactérie produisant de l'acide lactique qui permet de créer un environnement anaérobique favorable aux *Christensenellacées* telle qu'au moins une bactérie choisie parmi les bactéries du genre *Lactobacillus spp., Bifidobacterium spp., Streptococcus spp.* et/ou au moins un autre organisme favorisant les conditions anaérobiques nécessaires à la survie des *Christensenellacées* telle qu'au moins une levure choisie parmi des *Saccharomyces spp.* ou des microorganismes de la famille des *Methanobacteriaceae* et/ou
- au moins une bactérie associée à l'écosystème des *Christensenellacées* car elles facilitent leur survie dans l'intestin telle qu'au moins une bactérie choisie parmi les bactéries du phylum *Firmicutes, Bacteroidetes, Actinobacteria, Tenericutes,* et *Verrucomicrobia,* et/ou
- au moins une bactérie choisie parmi les bactéries de l'ordre des *Clostridales,* des *Verrucomicrobiales,* des *Aeromonadales,* des *Alteromonadales,* ML615J-28, RF32, YS2, de la famille des *Clostridiacées,* des *Lachnospiracées,* des *Ruminococcacées,* des *Bacteroidacées,* des *Enterococcacées,* des *Rikenéllacées,* des *Dehalobactériacées,* des *Veillonellacées* et/ou
- au moins une bactérie choisie parmi les bactéries du genre *Faecalibacterium, Akkermansia, Eubacterium* et *Oscillospira* telle que par exemple *Faecalibacterium prausnitzii, Akkermansia muciniphila, Eubacterium halii, Oscillospiraguilliermondii,* et/ou
- au moins un prébiotique tel que par exemple au moins un prébiotique choisi parmi les galactooligosaccharides, les fructooligosaccharides, les inulines, les arabinoxylans, les béta-glucanes, les lactoglobulines et/ou les béta-caséines, et/ou
- au moins un polyphénol tel que par exemple au moins un polyphénol choisi parmi la quercetin, le kaempferol, le resvératrol, les flavones (comme la lutéoline), les flavan- 3-ols (comme les catéchines), les flavanones (comme la narinénine), les isoflavones, les anthocyanidines, les proanthocyanidines, et/ou
- au moins un minéral et/ou au moins une vitamine et/ou au moins un agent nutritionnel, et/ou
- au moins un principe actif pharmaceutique présentant un effet dans la prévention et/ou le traitement de l'insuffisance rénale tel que par exemple la vitamine D, le sévélamer, le sulfonate de polystyrène sodique, le calcium, un agent calcimimétique (cinacalcet par exemple), un antihypertenseur, un diurétique, une statine, du fer et/ou un dérivé de l'érythropoïétine.

L'invention est à présent illustrée par des exemples de bactéries utiles selon l'invention, de procédés de cultures de ces bactéries, des exemples de compositions les contenant et des résultats d'essais démontrant l'efficacité des bactéries de la famille des *Christensenellacées* sur l'insuffisance rénale, en particulier sur l'insuffisance rénale chronique.

### EXEMPLES

### Exemple 1 : Christensenella minuta

Les bactéries *Christensenella minuta* peuvent-être cultivées selon le protocole opératoire décrit en suivant.
1/ Dissoudre un milieu RCM ("Reinforced Clostridial Medium" milieu clostridiale renforcé) déshydraté dans de l'eau distillée
2/Ajouter 0,5 mL/L de solution de résazurine-Na (0,1% p/v)
3/Porter à ébullition et refroidir à température ambiante tout en injectant un mélange gazeux à 80% de N₂ et à 20% de CO₂
4/Etaler le milieu sous la même atmosphère gazeuse dans des tubes de type Hungate anoxiques ou dans des flacons de sérum puis autoclaver
5/Avant utilisation, ajoutez 1,0 g de carbonate de sodium par litre à partir d'une solution mère anoxique stérile préparée avec un mélange gazeux à 80% de N₂ et à 20% de CO₂
6/ Vérifier le pH du milieu après autoclavage et ajuster le pH entre 7,3 et 7,5, en utilisant une solution mère anoxique stérile de bicarbonate de sodium (5% p / v) préparée dans une atmosphère gazeuse à 80% de N₂ et à 20% de CO₂.

### Exemple 2 : Christensenella massiliensis

Les bactéries *Christensenella massiliensis* peuvent-être cultivées selon le protocole opératoire décrit en suivant.
1/Préparer un milieu carboxyméthylcellulose (N₂ / CO₂) en suivant les instructions suivantes fournies par DSMZ (Deutsche Sammlung von Mikroorganismen und Zell- kulturen), présentées dans le Tableau 1.

**[Tableau 1]**

| | |
|---|---|
| Casitone | 30.0 g |
| Extrait de levure | 5.0 g |
| K₂HPO₄ | 5.0 g |
| Na-resazurin solution (0.1% w/v) | 0.5 ml |
| L-Cysteine-HCl × H2O | 0.5 g |
| D-Glucose | 4.0 g |
| Cellobiose | 1.0 g |
| Maltose | 1.0 g |
| Na₂CO₃ | 1.0 g |
| Filtrat de viande (voir Tableau 2) | 1000ml |

2/Dissoudre les différents constituants listés dans le tableau ci-dessus, sauf la cystéine, les carbohydrates et le carbonate.
3/Faire bouillir le milieu pendant 1 min, puis le laisser refroidir à la température ambiante sous une atmosphère gazeuse à 80% de N₂ et à 20% de CO₂.
4/Ajouter 0,5 g/L de L-cystéine-HCl × H₂O et le verser sous la même atmosphère gazeuse dans des tubes de type Hungate (pour les souches exigeant des particules de viande, introduire celles-ci en premier dans le tube, utiliser 1 partie de particules de viande pour 4 ou 5 parties de liquide).
5/ Autoclavage à 121°C pendant 20 min.
6/ Après autoclavage, ajouter le glucose, le cellobiose, le maltose et l'amidon provenant de solutions mères anoxiques stériles préparées à 100% de gaz N₂ et de carbonate à partir d'une solution mère anoxique stérile préparée sous des mélanges gazeux à 80% de N₂ et 20% de CO₂.
7/ Ajuster le pH du milieu à 7, si nécessaire.

La composition du filtrat de viande est présentée dans le Tableau 2.

**[Tableau 2]**

| | |
|---|---|
| Viande hachée (sans gras) | 500.0 g |
| NaOH 1N | 25.0 ml |
| Eau distillée | 1000ml |

Le filtrat de viande est préparé comme suit.
a/Utiliser du boeuf maigre ou de la viande de cheval.
b/Enlever la graisse et le tissu conjonctif avant de hacher.
c/ Mélanger la viande, l'eau et NaOH, puis faire bouillir pendant 15 min sous agitation.
d/ Laisser refroidir à la température ambiante, retirer la graisse de la surface et filtrer, en retenant les particules de viande et le filtrat.
e/Ajouter au filtrat de l'eau jusqu'à un volume final de 1000,0 ml.

Les bactéries doivent être cultivées en condition anaérobie à 37°C.

### Exemple 3 : Christensenella timonensis

Les bactéries *Christensenella timonensis* peuvent-être cultivées selon le même protocole opératoire que celui décrit à l'exemple 2 pour *Christensenella massiliensis.*

### Exemple 4 : Composition utile selon l'invention sous forme liquide

Un exemple de composition utile selon l'invention sous forme liquide est une composition comprenant *Christensenella minuta* 10⁹ CFU/mL dans le milieu de culture RCM anaérobie décrit ci-dessus modifié pour ne contenir aucun produit d'origine animale et enrichi en glycérol 5%.

La composition de l'exemple 4 est obtenue à partir d'une RCB («research cell bank » banque de cellules de recherche) préparée à base de *Christensenella minuta* 10¹⁰ CFU/ mL puis conservée congelée à -20°C dans un sachet hermétique à l'oxygène.

La composition congelée doit être réchauffée à température ambiante jusqu'à retrouver une forme liquide avant utilisation.

### Exemple 5 : Composition utile selon l'invention sous forme solide

Un exemple de composition utile selon l'invention sous forme lyophilisée peut être obtenu par lyophilisation de la composition de l'exemple 4 à l'état congelé.

### Essais démontrant l'efficacité de l'invention dans la prévention et le traitement de l'insuffisance rénale.

### Etude de l'effet de l'invention dans le traitement de l'insuffisance rénale in vitro

L'objectif de cette étude est de démontrer in vitro l'effet de bactéries de la famille des *Christensenellacées* dans traitement de l'insuffisance rénale. La démonstration a été réalisée sur deux des marqueurs de l'insuffisance rénale, notamment de l'insuffisance rénale chronique : l'homocitrulline et la dimethylarginine symétrique sérique.

La carbamylation est une modification post-traductionnelle non enzymatique de protéines qui est caractérisée par la liaison de l'acide isocyanique à des groupes amino de protéines (α-NH2 ou ε-NH2). Cette réaction entraîne la formation de produits dérivés de la carbamylation (CDP), le plus représentatif étant l'homocitrulline (HCit), qui est générée par la liaison de l'acide isocyanique au groupe ε-NH2 de la chaîne latérale des résidus de lysine.

L'homocitrulline est un produit dérivé de la carbamylation qui a été identifié comme un marqueur majeur de la morbidité et de la mortalité chez les patients atteints d'insuffisance rénale, notamment chronique. L'homocitrulline peut être absorbée dans l'intestin.

Le protocole opératoire de l'étude est décrit en suivant.
1/ Protocole de fermentation à partir de fèces d'origine humaine contenant *Christensenella spp .* :
   - Les donneurs ne devaient pas avoir pris d'antibiotiques durant les six mois précédents l'expérience et n'avoir aucun historique de désordres gastro-intestinaux. Les donneurs étaient âgés entre 18 et 60 ans.
   - La collecte des échantillons frais de leur fèces est obtenue dans des contenants stériles en plastique, conserves dans des flacons anaérobies contenant un sachet de 2,5l d'AnaeroGenTM d'OxoidTM (O₂ <0.1%; CO₂: 7-15%). Ces échantillons ont été apportés au laboratoire dans les deux heures après leur production.
   - Les échantillons de fèces ont été dilues au 1/5 (poids/volumes) dans une solution saline tamponnée au phosphate (1M) (PBS), pH 7,4. La suspension a été homogénéisée dans un stomacher pendant 120 secondes.
   - Milieu nutritif de base: le milieu nutritif de base a été préparé à partir de 2g/L de bouillon de tryptone de soja, 2g/L d'extrait de levure, 0,1g/L de NaCl, 0,04g/L de K₂HPO₄, 0,01g/L de MgSO₃.7H2O, 0,01g/L de CaCl₂.6H2O, 2g/L NaHCO₃, 0,5g/L de L-cystine HCl, 2mL/L de tween 80, 10µL/L de vitamine K1, 0,05g/L d'hème, 0,05g/L de sels biliaires, 4ml/L de résazarin (pH7)
   - Fermentation en bio fermenteur : Les bio fermenteurs de 20mL de contenance contenaient 18mL de milieu nutritif de base autoclavé (121°C pendant 15 minutes) et versé aseptiquement dans les bio fermenteurs stériles. Ce système a été laissé au repos toute la nuit avec un bullage d'azote sans oxygène à travers le milieu à un taux de 2mL/min. Le pH a été maintenu entre 6,7 et 6,9 en utilisant du HCl ou NaOH (0,5M). La température de chaque bio fermenteur était contrôlée à 37°C et le contenu du récipient homogénéisé avec un mélangeur magnétique
   - un mélange de protéines prédigérées (0,35g) a été ajouté dans les récipients avant l'inoculation avec 2mL d'inocula fécal à T0. Les protéines prédigérées ont été obtenues selon le protocole de digestion gastro-intestinale adapté de celui de Versantvoort et al (2005).
   - les échantillons ont été collectés avant la fermentation (T0) et après 48 heures de fermentation (T48), et congelés à -80°C jusqu'aux analyses.
2/ Quantification de l'homocitrulline et la dimethylarginine symétrique sérique :
   - 50 µL d'échantillons collectés et conservés à -80°C ont été mélangés avec 20 µL d'eau MilliQ contenant des standards internes.
   - Le mélange a été mélangé et filtré à travers un filtre de seuil 5-kDa pour retirer les macromolécules.
   - Les métabolites ont été détectés par analyses en électrophorèse capillaire- spectrométrie de masse à temps de vol (CE-TOFMS). La limite de détection des pics a été déterminée sur la base du ratio signal/bruit, S/N=3.
   Aire relative du pic = (aire du pic d'un métabolite)/(aire du pic du standard interne x quantité d'échantillon).
3/ Quantification de *Christensenella spp.*
   - L'ADN contenu dans les échantillons a été extrait en utilisant le kit NucleoSpin^{®}96 Soil de Macherey-Nagel selon les instructions du fabricant.
   - L'ADN extrait total a ensuite été fragmenté aléatoirement en fragments de 350 bp puis utilisé pour construire une librairie en utilisant le kit NEBNext Ultra Il par New England Biolabs selon les instructions du fabricant.
   - La librairie a ensuite été séquencée en utilisant du séquençage en paire de de 2 x 150 bp sur une plateforme Illumina HiSeq.
   - L'abondance des bactéries a été mesurée en créant un catalogue d'espèces métagénomiques (MGS) à partir d'un catalogue de référence contenant 22M de gènes. Ces MGS ont ensuite été associées à un niveau taxonomique adapté. Dans le cas des *Chrisi-ensenella,* celles-ci ont été détectées au niveau du genre et sont donc référencées dans cette expérience par *Christensenella spp.*

La quantité relative d'homocitrulline et diméthylarginine symétrique et l'abondance relative de *Christensenella spp.* ont été analysées et corrélées, obtenant une régression linéaire de R=-0,45 (n=18).

Les résultats sont présentés dans le Tableau 3.

**[Tableau 3]**

| **Echantillons** | **Abondance relative de *Christensenella* spp (×10⁻²)** | **Quantité relative de diméthylarginine symétrique sérique(×10⁻⁵)** | **Quantité relative de homocitrulline (×10⁻⁵)** |
|---|---|---|---|
| V1 | 7,55 | 0 | 2,87 |
| V2 | 3,18 | 0 | 0 |
| V3 | 8,19 | 0 | 4,18 |
| V4 | 2,63 | 0 | 3,50 |
| V5 | 1,26 | 0 | 2,92 |
| V6 | 2,87 | 0 | 3,47 |
| V7 | 7,20 | 3,78 | 4,34 |
| V8 | 2,91 | 4,21 | 18,82 |
| V9 | 6,32 | 0 | 0 |
| V10 | 1,21 | 5,10 | 39,15 |
| V11 | 4,23 | 5,92 | 19,14 |
| V12 | 1,49 | 4,61 | 14,81 |
| V13 | 9,83 | 0 | 0 |
| V14 | 4,12 | 0 | 6,14 |
| V15 | 6,45 | 0 | 0 |
| V16 | 2,02 | 0 | 7,56 |
| V17 | 5,23 | 0 | 11,77 |
| V18 | 7,57 | 0 | 7,68 |

On constate une corrélation négative entre les bactéries de la famille des *Christensenellacées* et l'homocitrulline et la diméthylarginine symétrique sérique, ce qui démontre un effet protecteur des bactéries de la famille des *Christensenellacées* contre l'insuffisance rénale.

Ainsi les bactéries de la famille des *Christensenellacées* sont capables d'agir en diminuant la production de marqueurs de l'insuffisance rénale, notamment d'homocitrulline et de diméthylarginine symétrique sérique. Elles peuvent donc être utilisées pour prévenir et/ou traiter l'insuffisance rénale, aiguë ou chronique.

## Revendications

1. Bactérie de la famille des *Christensenellacées* pour son utilisation dans la prévention et/ou le traitement de l'insuffisance rénale chez l'être humain ou l'animal.

2. Bactérie de la famille des *Christensenellacées* pour son utilisation selon la revendication 1, chez des êtres humains ou des animaux présentant une insuffisance rénale corrélée à une hyperproduction d'homocitrulline et/ou de dimethylarginine symétrique sérique.

3. Bactérie de la famille des *Christensenellacées* pour son utilisation selon l'une des précédentes revendications, *Christensenellacées* pour son utilisation dans la prévention et/ou le traitement de l'insuffisance rénale chronique chez l'être humain ou l'animal.

4. Bactérie de la famille des *Christensenellacées* pour son utilisation selon l'une des précédentes revendications, **caractérisée en ce que** ladite bactérie est une bactérie du genre *Christensenella.*

5. Bactérie de la famille des *Christensenellacées* pour son utilisation selon l'une des précédentes revendications, **caractérisée en ce que** ladite bactérie est choisie parmi *Christensenella massiliensis, Christensenella timonensis et Christensenella minuta.*

6. Composition comprenant au moins une bactérie de la famille des *Christensenellacées,* pour son utilisation dans la prévention et/ou le traitement de l'insuffisance rénale chez l'être humain ou l'animal.

7. Composition pour son utilisation selon la revendication 6, dans la prévention et/ou le traitement de l'insuffisance rénale chronique chez l'être humain ou l'animal.

8. Composition pour son utilisation selon la revendication 6 ou 7, **caractérisée en ce qu'**elle se présente sous forme liquide ou solide.

9. Composition pour son utilisation selon la revendication 8, **caractérisée en ce que** les bactéries sous forme solide sont présentes sous forme lyophilisée.

10. Composition pour son utilisation selon l'une des revendications 6 à 9, **caractérisée en ce que** les bactéries présentes sont pour au moins 50% des bactéries vivantes (en nombre).

11. Composition pour son utilisation selon l'une des revendications 6 à 10, par voie orale, rectale ou inhalée.

12. Composition pour son utilisation selon l'une des revendications 6 à 11, **caractérisée en ce qu'**elle se présente sous forme de poudre, de poudre microencapsulée, de gélule, de capsule, de comprimé, de pastille, de granulés, d'émulsion, de suspension ou de suppositoire.

13. Composition pour son utilisation selon l'une des revendications 6 à 12, **caractérisée en ce qu'**elle se présente sous une forme gastro-résistante.

14. Composition pour son utilisation selon l'une des revendications 6 à 13, **caractérisée en ce qu'**elle comprend au moins un probiotique et/ou au moins un prébiotique.

15. Composition pour son utilisation selon l'une des revendications 6 à 14, **caractérisée en ce qu'**elle comprend également :
- au moins une bactérie produisant de l'acide lactique et/ou au moins un autre organisme favorisant les conditions anaérobiques nécessaires à la survie des *Christensenellacées,* et/ou
- au moins une bactérie associée à l'écosystème des *Christensenellacées,* et/ou
- au moins une bactérie choisie parmi les bactéries du genre *Faecalibacterium, Akkermansia, Eubacterium et Oscillospira,* et/ou
- au moins un polyphénol, et/ou
- au moins un minéral et/ou au moins une vitamine et/ou au moins un agent nutritionnel, et/ou
- au moins un principe actif pharmaceutique présentant un effet de prévention ou de traitement de l'insuffisance rénale.

## Patentansprüche

1. Bakterium der Familie der *Christensenellaceae* zur Verwendung bei der Prävention und/oder der Behandlung von Niereninsuffizienz bei Menschen oder Tieren.

2. Bakterium der Familie *Christensenellaceae* zur Verwendung nach Anspruch 1 bei Menschen oder Tieren, die eine Niereninsuffizienz aufweisen, die mit einer Hyperproduktion von Homocitrullin und/oder symmetrischem Dimethylarginin im Serum einhergeht.

3. Bakterium der Familie der *Christensenellaceae* zur Verwendung nach einem der vorstehenden Ansprüche, *Christensenellaceae* zur Verwendung bei der Prävention und/oder der Behandlung von chronischer Niereninsuffizienz bei Menschen oder Tieren.

4. Bakterium der Familie der *Christensenellaceae* zur Verwendung nach einem der vorstehenden Ansprüche, **dadurch gekennzeichnet, dass** das Bakterium ein Bakterium der Gattung *Christensenella* ist.

5. Bakterium der Familie der *Christensenellaceae* zur Verwendung nach einem der vorstehenden Ansprüche, **dadurch gekennzeichnet, dass** das Bakterium ausgewählt ist aus *Christensenella massiliensis, Christensenella timonensis* und *Christensenella minuta.*

6. Zusammensetzung, umfassend mindestens ein Bakterium der Familie der *Christensenellaceae,* zur Verwendung bei der Prävention und/oder der Behandlung von Niereninsuffizienz bei Menschen oder Tieren.

7. Zusammensetzung zur Verwendung nach Anspruch 6 bei der Prävention und/oder der Behandlung von chronischer Niereninsuffizienz bei Menschen oder Tieren.

8. Zusammensetzung zur Verwendung nach Anspruch 6 oder 7,
**dadurch gekennzeichnet, dass** sie in flüssiger oder fester Form vorliegt.

9. Zusammensetzung zur Verwendung nach Anspruch 8,
**dadurch gekennzeichnet, dass** die Bakterien in fester Form in lyophilisierter Form vorliegen.

10. Zusammensetzung zur Verwendung nach einem der Ansprüche 6 bis 9, **dadurch gekennzeichnet, dass** die vorliegenden Bakterien zu mindestens 50 % als lebende Bakterien (nach Anzahl) vorliegen.

11. Zusammensetzung zur Verwendung nach einem der Ansprüche 6 bis 10 auf einem oralen, rektalen oder inhalierten Weg.

12. Zusammensetzung zur Verwendung nach einem der Ansprüche 6 bis 11, **dadurch gekennzeichnet, dass** sie in Form eines Pulvers, eines mikroverkapselten Pulvers, einer Hartkapsel, einer Kapsel, einer Tablette, einer Pastille, von Granulaten, einer Emulsion, einer Suspension oder eines Suppositoriums vorliegt.

13. Zusammensetzung zur Verwendung nach einem der Ansprüche 6 bis 12, **dadurch gekennzeichnet, dass** sie in magensaftresistenter Form vorliegt.

14. Zusammensetzung zur Verwendung nach einem der Ansprüche 6 bis 13, **dadurch gekennzeichnet, dass** sie mindestens ein Probiotikum und/oder mindestens ein Präbiotikum umfasst.

15. Zusammensetzung zur Verwendung nach einem der Ansprüche 6 bis 14, **dadurch gekennzeichnet, dass** sie zudem umfasst:
- mindestens ein Milchsäure produzierendes Bakterium und/oder mindestens einen anderen Organismus, der anaerobe Bedingungen, die für das Überleben der *Christensenellaceae* erforderlich sind, fördert, und/oder
- ein Bakterium, das mit dem Ökosystem der *Christensenellaceae* verbunden ist, und/oder
- mindestens ein Bakterium, ausgewählt aus den Bakterien der Gattung *Faecalibacterium, Akkermansia, Eubacterium* und *Oscillospira,* und/oder
- mindestens ein Polyphenol, und/oder
- mindestens ein Mineral und/oder mindestens ein Vitamin und/oder mindestens einen Nährstoff, und/oder
- mindestens einen pharmazeutischen Wirkstoff, der eine Wirkung zur Prävention oder Behandlung von Niereninsuffizienz aufweist.

## Claims

1. Bacteria from the *Christensenellaceae* family for use in the prevention and/or treatment of renal insufficiency in humans or animals.

2. Bacteria from the *Christensenellaceae* family for use according to claim 1 in humans or animals presenting renal insufficiency correlated with hyperproduction of homocitrulline and/or serum symmetric dimethylarginine.

3. Bacteria from the *Christensenellaceae* family for use according to either of the preceding claims, *Christensenellaceae* for use in the prevention and/or treatment of chronic renal insufficiency in humans or animals.

4. Bacteria from the *Christensenellaceae* family for use according to any of the preceding claims, **characterized in that** said bacteria is bacteria of the genus *Christensenella.*

5. Bacteria from the *Christensenellaceae* family for use according to any of the preceding claims, **characterized in that** said bacteria is selected from *Christensenella massiliensis, Christensenella timonensis and Christensenella minuta.*

6. Composition comprising at least one bacterium from the *Christensenellaceae* family for use in the prevention and/or treatment of renal insufficiency in humans or animals.

7. Composition for use according to claim 6 in the prevention and/or treatment of chronic renal insufficiency in humans or animals.

8. Composition for use according to claim 6 or 7, **characterized in that** it is in liquid or solid form.

9. Composition for use according to claim 8, **characterized in that** the bacteria in solid form are present in lyophilized form.

10. Composition for use according to any of claims 6 to 9, **characterized in that** at least 50% (by number) of the bacteria present are living bacteria.

11. Composition for use according to any of claims 6 to 10 for administration orally, rectally or by inhalation.

12. Composition for use according to any of claims 6 to 11,
**characterized in that** it is in the form of powder, microencapsulated powder, gelcaps, capsules, tablets, pastilles, granules, emulsion, suspension or suppositories.

13. Composition for use according to any of claims 6 to 12,
**characterized in that** it is gastro-resistant.

14. Composition for use according to any of claims 6 to 13,
**characterized in that** it comprises at least one probiotic and/or at least one prebiotic.

15. Composition for use according to any of claims 6 to 14,
**characterized in that** it also comprises:
- at least one lactic-acid-producing bacteria and/or at least one other organism promoting the anaerobic conditions required for the survival of *Christensenellaceae,* and/or
- at least one bacterium associated with the *Christensenellaceae* ecosystem, and/or
- at least one bacterium selected from the genus *Faecalibacterium, Akkermansia, Eubacterium and Osclospira,* and/or
- at least one polyphenol, and/or
- at least one mineral and/or at least one vitamin and/or at least one nutritional agent, and/or
- at least one active pharmaceutical ingredient providing an effect of preventing or treating renal insufficiency.
